# EUROPEAN PATENT APPLICATION

(11) **EP 3 725 343 A1**
(43) Date of publication of application: **21.10.2020**
(21) Application number: 18888211.2
(22) Date of filing: 10.12.2018
(51) Int. Cl.: A61M 3/02

(54) **DISPOSABLE DEVICE FOR EAR WASHING**

(30) Priority: 11.12.2017 ES 201731501 U
(71) Applicant: Servicio Andaluz de Salud, 41071 Sevilla (ES)
(72) Inventor: TOMÁS ROJAS, José, 41071 Sevilla (ES)
(74) Representative: ABG Intellectual Property Law, S.L.
(86) International application number: PCT/ES2018/070793
(87) International publication number: WO 2019/115853

(57) **Abstract**

The invention relates to a disposable device for ear washing, which comprises a disposable syringe and a disposable cone connectable to the distal end of the syringe.

## Description

### OBJECT OF THE INVENTION

The present invention belongs to the field of nursing-medicine, and more particularly to the field of otorhinolaryngology.

The object of the present invention is a new device which allows performing ear washing on a patient in a cleaner, simpler, more hygienic, comfortable, and cost-effective manner.

### BACKGROUND OF THE INVENTION

Ear washing is part of the daily clinical practice of nursing staff in healthcare centers. The method of cleaning the ear is performed by means of injecting pressurized warm water using means suitable to that end. Although a syringe particularly designed for this task, referred to as the Reiner-Alexander syringe, is available, alternative solutions such as the use of a syringe connected to a cut venous catheter or a syringe connected to a cut butterfly-type venous catheter, are often used. These elements are described in more detail below.

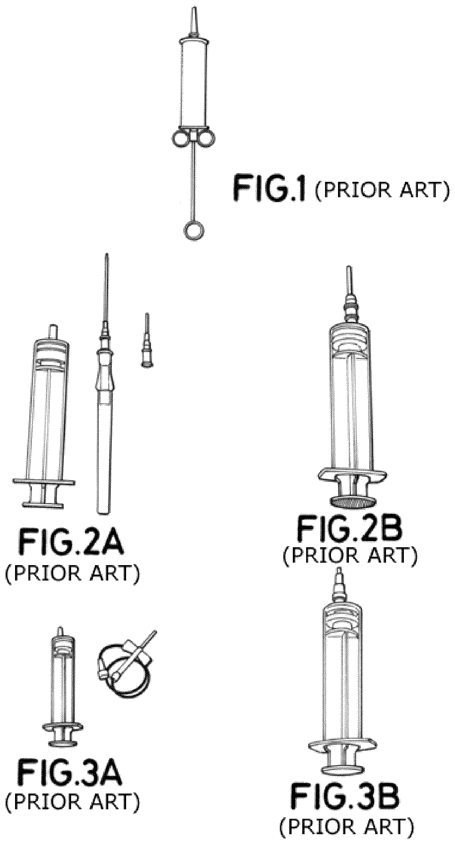

The Reiner-Alexander syringe, shown in Figure 1, is a metal syringe which can have a volume of about 100 cc and an interchangeable cone. The use of this syringe requires applying force on the plunger so that the water comes out at a pressure that is sufficient to perform an effective cleaning. The plunger is manually actuated through rings configured for receiving the thumb, the index finger, and the middle finger of the health professional. Between 1 and 3 irrigations, with a total volume of approximately 300 cc, are usually required for an ear cleaning process.

This syringe, which has been used since the 19^{th} century, has some drawbacks. The large size of the syringe causes significant difficulties for health professionals in general, and for those having small hands in particular. Moreover, at times part of the water tends to come out through the area of the plunger, splashing on the professional, floor, and patient. Furthermore, the cleaning, disinfection, and maintenance of the material constitutes a significant investment in terms of time and hygienic material, and even then suitable asepsis is not achieved.

To solve some of these problems, disposable elements modified to adapt conventional syringes for use in this context are often utilized. Specifically, a conventional syringe with a Luer-gauge cone which is connected to an additional element for increasing the length of the cone and reducing its diameter is sometimes used. This additional element can be, for example, a cut 14G, 16G, or 18G venous catheter, as seen in Figures 2a and 2b. The connection of a cut butterfly-type venous catheter, as seen in Figures 3a and 3b, is also frequently used. In any of the cases, time is lost for forming the system. Furthermore, in these solutions the need to cut the butterfly connection or the catheter implies reduced safety if the cut is not made at a suitable distance and plane. Moreover, the portion of the butterfly connection or catheter that is not used must be disposed of, which involves a high cost.

Therefore, there is still a need in this field for devices which allow performing ear cleaning in a cleaner, simpler, more hygienic, comfortable, and cost-effective manner.

### DESCRIPTION OF THE INVENTION

The present invention solves the preceding problems thanks to a new device formed by a conventional disposable syringe provided with a cone having a greater length and smaller diameter in comparison with the cones of conventional syringes. In fact, a conventional syringe, for example a Luer syringe, is not suitable for carrying out this method fundamentally due to two reasons: the cone is not long enough for it to be introduced sufficiently in the outer ear canal and oriented in the suitable direction; and the outlet diameter of the cone is too large for the required pressures to be obtained manually. The present invention solves those problems by providing an essentially conventional syringe with a cone having suitable length and diameters for performing an ear cleaning method. This new device is advantageous in relation to the devices used up until now for different reasons.

First, with respect to the 100 cc Reiner-Alexander syringe, this new device is advantageous because it is made of disposable materials, which increases patient safety and eliminates the time required for cleaning and disinfection. Furthermore, the use of a conventional syringe makes it easier for health professionals in general, and for those with small hands in particular, to be able to carry out the method without major problems. The use of a conventional syringe also prevents water from coming out through the plunger, so the cleanliness of the method is increased.

Secondly, with respect to the syringe connected to a venous catheter or the like, this new device is advantageous because it reduces the handling required for manufacturing same, which thereby allows saving time. Furthermore, making cuts on the catheter with planes that may damage the outer ear canal or may not have the suitable distance is prevented. Furthermore, the cost of this new device is considerably lower than the cost of a conventional syringe plus the cost of the cut venous catheter.

The disposable device for ear washing of the present invention fundamentally comprises a disposable syringe and a disposable cone connectable to the distal end of the syringe. Each of these elements is described in greater detail below.

### a) Syringe

It is a conventional disposable syringe formed by a cylindrical reservoir along which a plunger moves. The reservoir has a distal outlet hole, such that a forward movement of the plunger causes the liquid contained in the reservoir to come out through said distal outlet hole.

Regarding the volume of the syringe, the inventor of the present device has checked that in order to perform an ear cleaning process with the device of the invention, injection of a volume of water between about 40 cc and 140 cc is required. Taking this into account, the volume of the reservoir of the syringe of the device of the invention has been selected such that the cleaning requires the least number of injections but always assuring that the size of the syringe does not entail a handling problem for the professionals. Therefore, the volume of the reservoir of the syringe of the device of the present invention is between 20 cc and 30 cc, more preferably between 25 cc and 30 cc. This volume means that a cleaning method can be performed with between 2 and 7 irrigations, which is a reasonable number, and at the same time prevents handling problems for health professionals.

### b) Cone

It is a disposable cone that is connectable to the distal end of the syringe to allow the liquid to come out driven by the plunger.

The length of the cone is between 2.5 cm and 3.5 cm, more preferably between 2.8 cm and 3.2 cm. It has been checked that this length is ideal for the introduction and orientation thereof in the outer ear canal of the patient during the cleaning method. This length is in contrast with the length of a conventional syringe, ranging between 1 cm and 1.5 cm, which is clearly not sufficient for suitably performing an ear cleaning method.

The diameter of the cone at its outlet end is between 1.5 mm and 1.75 mm, more preferably between 1.6 mm and 1.7 mm. This outlet diameter, smaller than the diameter of a conventional Luer-gauge syringe or a Reiner-Alexander syringe, allows the professional to easily achieve an output pressure sufficient for ear cleaning by means of manually actuating the syringe.

In a preferred embodiment of the invention, the cone and the syringe can be independent parts connected in a separable manner. For example, a cone with the described length and diameter is connected to the distal end of a conventional syringe having a suitable volume. These two parts, syringe and cone, may come connected or not connected in the same sterile wrap. More preferably, the cone may be a Luer-to-Record-type connector about 3 cm in length. In fact, a Luer-to-Record-type connector is a part about 3 cm in length designed to modify the outlet cone of a syringe from Luer gauge to Record gauge. Luer gauge refers to an inner diameter of 1.85 mm, whereas Record gauge refers to an inner diameter of 1.74 mm. Therefore, when a Luer-to-Record connector like the one described is fixed to the distal end of a conventional syringe, said conventional syringe is provided with a cone having suitable dimensions both in length and in diameter for ear washing.

In a preferred embodiment alternative to the foregoing, the cone and the syringe can be attached forming a single part. That is, in this case the syringe is formed in an integral manner with the cone having the described length and diameter.

The device of the invention, including both the syringe and the cone, can be manufactured from any disposable material commonly used in the medical field. For example, it may be made of plastic or the like.

Therefore, the described device solves the preceding problems as it makes it easier for health professionals to perform ear cleaning, prevents the need to clean metal devices such as the Reiner-Alexander syringe, and is furthermore highly cost-effective in comparison with the *"handmade"* devices used today. Furthermore, the hygiene of the method is increased.

### BRIEF DESCRIPTION OF THE FIGURES

Figure 1 shows a Reiner-Alexander syringe according to the prior art.
Figures 2a and 2b show, respectively, the starting materials and the resulting device of a first type of *"handmade"* device of the prior art.
Figures 3a and 3b show, respectively, the starting materials and the resulting device of a second type of *"handmade"* device of the prior art.
Figure 4 shows a first example of the device according to the present invention.
Figures 5a and 5b show, respectively, the starting materials and the resulting device of a second example of the device according to the present invention.

### PREFERRED EMBODIMENT OF THE INVENTION

The invention is described below in reference to the attached figures.

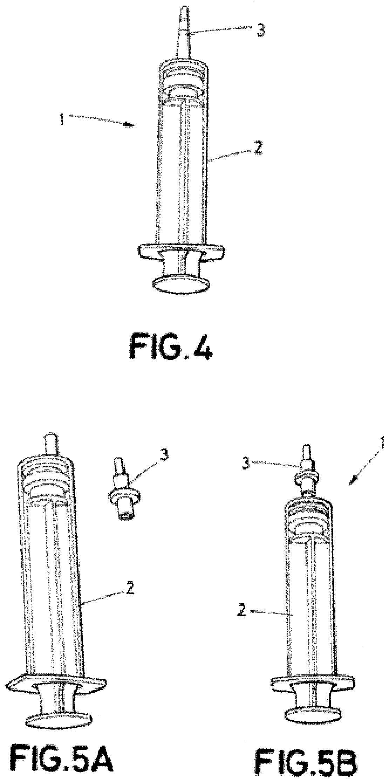

Figure 4 shows a first example of the device (1) formed by a disposable syringe (2) with a volume of 25 cc provided with a cone (3) at its proximal end such that both the syringe and the cone form a single part. In this example, the cone (3) has a length of 3 cm and an inner diameter that varies from 2 mm in its base, located right at the distal end of the reservoir of the syringe (2), to 1.5 mm at its tip, constituting the distal end of the device (1).

Figure 5a shows the elements used for forming a second example of the device (1) according to the present invention. In this second example, the syringe (2) and the cone (3) are independent elements which are connected to one another to form the device (1).

The first element is a conventional disposable 20 cc Luer syringe (2). As mentioned hereinabove, a Luer-gauge syringe (2) is in itself not suitable for performing ear cleaning because the outlet diameter of the Luer cone, 1.85 mm in diameter, is too large to allow manually generating sufficient pressure, and furthermore it is too short to enable the health professional to suitably orient same inside the ear.

The second element is a Luer-to-Record connector (3). The Luer-to-Record connector (3) is a part designed to modify the outlet diameter of the cone of a syringe from Luer gauge to Record gauge, 1.74 mm in diameter. Furthermore, this Luer-to-Record connector (3) has a length of about 3 cm, which allows increasing the length of the conventional Luer cone of the syringe (2), about 1 cm, to said length. The Luer-to-Record connector (3) is configured to be coupled by pressure, in a separable manner, to the cone of the Luer syringe (2). The Luer-to-Record connector is also disposable.

Figure 5b shows the Luer-to-Record connector (3) already fixed to the distal end of the Luer syringe (2). The device (1) that is created is simple and cost-effective, allowing any health professional to easily perform ear cleaning on the patient, regardless of the size of his or her hands and particular skill in holding the syringe.

## Claims

1. A disposable device (1) for ear washing, **characterized in that** it comprises:
- a disposable syringe (2) having a volume between 20 cc and 30 cc; and
- a disposable cone (3) connectable to the distal end of the syringe (2), wherein the cone (3) has a length between 2.5 cm and 3.5 cm and a diameter between 1.5 mm and 1.75 mm.

2. The disposable device (1) according to claim 1, wherein the cone (3) is a Luer-to-Record-type connector about 3 cm in length.

3. The disposable device (1) according to claim 1, wherein the cone (3) and the syringe (2) are attached forming a single part.

4. The disposable device (1) according to any of the preceding claims, wherein the volume of the syringe is between 25 cc and 30 cc.

5. The disposable device (1) according to any of the preceding claims, wherein the length of the cone (3) is between 2.8 cm and 3.2 cm.

6. The disposable device (1) according to any of the preceding claims, wherein the diameter of the cone (3) is between 1.6 mm and 1.7 mm.

7. The disposable device (1) according to any of the preceding claims which is made of plastic.
